# EUROPEAN PATENT APPLICATION

(11) **EP 1 873 167 A2**
(43) Date of publication of application: **02.01.2008**
(21) Application number: 06725794.9
(22) Date of filing: 15.02.2006
(51) Int. Cl.: C08B 37/16, A61K 47/38, A61K 47/40

(54) **METHOD OF OBTAINING HYDROGELS OF CYCLODEXTRINS WITH GLYCIDYL ETHERS, COMPOSITIONS THUS OBTAINED AND APPLICATIONS THEREOF**

(30) Priority: 25.02.2005 ES 200500556
(71) Applicant: UNIVERSIDADE DE SANTIAGO DE COMPOSTELA, 15782 Santiago de Compostela (ES)
(72) Inventor: ALVAREZ LORENZO, Carmen, E-15782 Santiago de Compostela (ES); RODRÍGUEZ-TENREIRO SÁNCHEZ, Carmen, E-15782 Santiago de Compostela (ES); TORRES LABANDEIRA, Juan José, E-15782 Santiago de Compostela (ES); CONCHEIRO NINE, Angel, E-15782 Santiago de Compostela (ES)
(74) Representative: Carvajal y Urquijo, Isabel
(86) International application number: PCT/ES2006/000096
(87) International publication number: WO 2006/089993

(57) **Abstract**

The invention relates to a method of obtaining hydrogels based on (i) cyclodextrins, (ii) cyclodextrins and cellulose ethers or (iii) cyclodextrins and guar gum or the derivatives of same, using molecules containing two or more glycidyl ether groups in the structure thereof as a cross-linking agent. The invention also relates to the compositions thus obtained, which can include active substances and pharmaceuticals and which form inclusion complexes with cyclodextrins. The invention further relates to the use of same as components of controlled-release devices, such as transdermal pharmaceuticals forms, vaginal, otic, ocular, rectal, oral or buccal transmucosal forms and parenteral implants, which are designed to administer active substances of pharmaceuticals to humans, animals or plants, or as components of cosmetic formulations. In addition, the invention relates to the use of said compositions as chelating agents in the extraction of biological or toxic molecules from living organisms or contaminating substances form water.

## Description

### Field of the Invention

The present invention relates to a process for obtaining hydrogels formed by: i. cyclodextrins or their derivatives; or ii. cyclodextrins or their derivatives, and cellulose ethers or their derivatives; or iii. cyclodextrins or their derivatives, and guar gums or their derivatives; iv. and, furthermore, using as a crosslinking agent: molecules containing in their structure two or more glycidyl ether groups; the compositions obtained by this process; and the use and applications of the compositions in the preparation of dosage forms, medicinal products, plant protection products, sequestering agents and cosmetic products.

### State of the Art

In recent decades, along with the continuous improvement of immediate-release dosage forms, a growing interest has arisen in the development of more complex formulations that can prolong the drug delivery process, maintaining therapeutic levels for long periods of time (first generation of controlled delivery systems), specifying the moment in which the delivery is to commence (second generation of controlled delivery systems) or even providing a delivery rate regulated by the presence of a certain substance, the concentration of which is a function of the degree to which the organ or tissue is affected (third generation of controlled delivery systems) (Chien and Lin, Clin. Pharmacokinet. 41: 1267-1299, 2002). The current concept of a safe and effective dosage form also includes, in certain circumstances, the need for the drug to be released into the most suitable tissue or organ. These approaches are able to optimize the pharmacological effect, reduce the side effects, simplify treatments and improve therapeutic compliance.

The practical materialization of these dosage forms of drugs has been able to become a reality thanks to the development of new polymer materials, some of which are able to modify their formation as a response to changes in the biological or physical-chemical characteristics of the medium, whereby they are referred to as "smart" materials. The incorporation of these polymer materials into some classic dosage forms -gels, particles, tablets- give them new functions, considerably broadening their potential to be applied in the field of biomedicine (Yuk and Bae, Crit. Rev. Ther. Drug Carrier Syst. 16: 385-423, 1999).

The high affinity for water and the versatility of obtainment processes which lead to the formation of the three-dimensional structure characterizing hydrogels crosslinked by physical or chemical processes make them very useful for designing formulations with mechanical and drug delivery properties suitable for the requirements for virtually all administration routes. The huge potential of hydrogels in the field of pharmaceutical technology is clearly demonstrated by their numerous applications in the treatment of ocular pathologies and in the oral, transdermal, ocular, nasal, rectal or vaginal administration of drugs in both human and veterinary medicine (Peppas et al., Eur. J. Pharm. Biopharm. 50: 27-46, 2000; Rathbone et al., Controlled Release Veterinary Drug Delivery, Elsevier, Amsterdam, 2000). Hydrogels also have interesting applications as active substance and nutrient release systems components for plants (Osada and Kajiwara, Gels Handbook, vol. 3, Chapter 5, Academic Press, San Diego, 2001, pp. 259-285), as trapping systems for capturing toxic substances or biological molecules in living organisms (Sellergren et al., Chem. Mater. 10: 4037-4046, 1998) and as contaminating substance sequestrants in the treatment of water (Sanbe et al., Analyt. Sci. 19: 715-719, 2003).

The properties of the co-monomers and of the polymers used in hydrogel synthesis, together with their relative proportion and the degree of crosslinking, are determining factors of the properties of the resulting system. Therefore, these critical variables must be defined for each specific application (Eichembaum et al., Macromolecules 32: 4867-4878, 1999).

A very interesting approach to promote the incorporation of the drug and to modulate its delivery consists of optimizing the affinity of some of the components of the polymer lattice for the drug. The system thus incorporates the necessary dose and the delivery is controlled by the changes in the affinity of the drug for the chemical groups involved in its incorporation to the hydrogel. The changes are induced by the alterations in the physical-chemical characteristics of the medium or by the competitive binding of a physiological substance. These approaches are a very useful resource for achieving that the release occurs in specific places or for regulating the delivery rate by feedback mechanisms. A system with these features has recently been described based on N-isopropylacrylamide hydrogels (Alvarez-Lorenzo and Concheiro, J. Controlled Rel. 80: 247-257, 2002).

The therapeutic usefulness of "smart" polymers may considerably increase if the creation, in their internal structure, of receptors recognizing specific molecules is promoted (Alvarez-Lorenzo et al., Macromolecules 33: 8693-8697, 2000; Alvarez-Lorenzo and Concheiro, *op. cited* 2002). The difficulty surrounding the preparation of polymers having good molecular recognition capacity in an aqueous medium involves a serious drawback for applying this approach in the field of biomedicine given that in an aqueous environment, electrostatic-type interactions or interactions due to hydrogen bridges are less intense and hydrophobic reactions are less selective than in an organic medium. To palliate these limitations, processes aimed at incorporating cyclodextrins into the structure of hydrogels either by simple physical mixing or by forming part of covalently bonded monomer derivatives have been developed (Asanuma et al., Adv. Mater. 12: 1019-1030, 2000).

Cyclodextrins are toroidal-shaped cyclic oligomers formed by glucopyranose units, having a hydrophobic character in their inner part and a hydrophilic character in their outer surface. Greek letters are used to indicate the number of α-D-glucose units a cyclodextrin contains; for example, α- (6 units), β- (7 units) or y-cyclodextrin (8 units). This notation system is also used to designate other cyclodextrins made up of more than eight α-1,4-glucopyranose units, known as large cyclodextrins. Natural cyclodextrins have a variable number of hydroxyl groups through which different functional groups can be incorporated to give rise to a large variety of derivatives (Dûchene and Wouessidjewe, Pharm. Technol. 14: 22-30, 1990). These new groups in turn provide characteristic binding sites and modify their physical-chemical properties, providing them specific functions. To prepare substituted cyclodextrin derivatives, molecules with epoxide groups, such as for example 1,4-butanediol diglycidyl ether to obtain trihydroxy-dioxadodecyl-beta-cyclodextrin or propylene oxide to obtain hydroxypropyl-beta-cyclodextrin (US patents 4,596,795 and 4,727,064; Pitha and Pitha, J. Pharm. Sci. 74: 987-990, 1985), can be used.

One of the most interesting properties with the most practical importance of cyclodextrins is their capacity to form inclusion complexes with a wide variety of molecules. In order to be able to form these complexes, in addition to being less polar than water, the molecules must be able to be partially or completely introduced into the cyclodextrin cavity (Uekama, Chem. Pharm. Bull. 52: 900-915, 2004). The energetic state of the water molecules located inside the cavity is unfavorable as a result of the repulsions formed between their polar groups and the apolar groups of cyclodextrin. The substitution of water molecules with molecules, or with parts of molecules, of other less polar substances leads to the formation of an inclusion complex (Szejtli, Cyclodextrin inclusion complexes. In Cyclodextrin Technology (Szejtli, Ed.) Kluwer Academic Publishers, Budapest, 1988, p. 85). As a result of the variety of the diameters of their cavities, cyclodextrins can form inclusion complexes with a wide range of molecules with very different sizes (Fundueanu et al., J. Chromatogr. B 791: 407-419, 2003). Complex formation is a dynamic process in which permanent bonds (covalent or ionic) are not generated between the guest and the host, and in which only hydrophobic or van der Waals-type interaction forces are involved. Both the formation and the dissociation of the complexes occur very quickly. As a result, although they are usually represented as a single species, they are dynamic systems in which cyclodextrin and the free active ingredient coexist in dissolution with the complex itself (Uekama, *opus cited* 2004). Complex formation can be shown by different, relatively simple, instrumental techniques, such as ultraviolet-visible spectrophotometry (Fundueanu et al., J. Chromatogr. B 791: 407-419, 2003), the Raman spectroscopy (Iliescu et al., Eur. J. Pharm. Sci. 22: 487-495, 2004), X-ray spectroscopy and nuclear magnetic resonance (NMR) spectroscopy, or calorimetric techniques.

The size and shape of the guest molecule are determining factors so that a complex is formed with a certain cyclodextrin. The suitability of the dimensions of the guest molecule to that of the cyclodextrin cavity conditions the value of the complex formation constant, the high values of this constant indicating that the interaction between the two species is intense (Perlovich et al., Eur. J. Pharm. Sci. 20: 197-200, 2003). The 1:1 stoichiometric drug:cyclodextrin ratio is the most frequent ratio, although complexes with ratios of 1:2, 1:3, 2:3 or even 3:2 have also been described (Endo et al., Chem. Pharm. Bull. 45: 1856-1859, 1997). If the guest molecule is very bulky, as occurs for example in the case of steroidal hormones, liposoluble vitamins or cardiotonic glycosides, the inclusion complex formation may require more than one cyclodextrin molecule for each drug molecule. The polarity of the guest molecule also affects the complex formation process, conditioning its affinity for the cavity and the orientation therein. The arrangement that the drug adopts is generally the arrangement provided by the maximum possible contact between its hydrophobic part and the inner surface of the cavity. For similar molecular dimensions, the affinity for the cavity is greater the more hydrophobic the drug is (Saenger, Angew. Chem. Int. Ed. Eng. 92: 343-361, 1980).

Modifications in the physical-chemical properties of drugs (solubility, stability and volatility) derived from their incorporation to inclusion complexes with cyclodextrins are used as a basis for numerous pharmaceutical applications (Nakai et al. Chem. Pharm. Bull. 31:3745-3747, 1983; Loftsson and Brewster, J. Pharm. Sci. 85:1017-1025, 1996; Loftsson et al. Am. J. Drug Del. 2: 261-275, 2004). Cyclodextrins are also used as functional drug carriers to control the delivery rate from different dosage forms (Uekama, *op. cited* 2004). The more hydrophilic varieties are suitable for accelerating the delivery rate of liposoluble drugs from solid forms and improving their bioavailability. For this reason they are very useful as excipients for immediate-release forms.

A process for obtaining solid forms with cyclodextrins for the accelerated release of active ingredients is described in European patent application 00916925.1 (Spanish patent ES 2187457 of Berndl et al.). Hydrophobic cyclodextrins can be used as carriers of hydrosoluble drugs, for example peptides and proteins, in prolonged delivery forms. Certain enteric cyclodextrins, such as O-carboxymethyl-O-ethyl-β-cyclodextrin, are useful for preparing delayed delivery systems. The combination of different cyclodextrins and/or pharmaceutical additives allows obtaining formulations combining suitable oral bioavailability with prolonged therapeutic effects (Bibby et al., Int J. Pharm. 197: 1-11, 2000).

The incorporation of cyclodextrins into systems containing high proportions of polymers (polymer matrices), in which the chains are free (physically interlinked or non-crosslinked) or covalently bonded (chemically crosslinked), allows modifying the drug release mechanism. When cyclodextrins are added as free components, the solubility and diffusivity of the drug is modified, hydration of the matrix is aided and erosion is promoted (Bibby et al, Int. J. Pharm. 197: 1-11, 2000; Pose-Vilarnovo et al. J. Controlled Rel., 94, 351-363, 2004). The interaction of cyclodextrin with the polymer forming the matrix can significantly alter its capacity to form inclusion complexes with drugs. The presence of hydrophilic polymers in low proportions in the medium generally aids the process (Loftsson et al., Int. J. Pharm. 110: 169-177, 1995). The solubility of the complex and its capacity to diffuse towards the outside of the matrix are determining factors of the delivery rate of drugs in this type of systems. Cyclodextrins themselves can behave as crosslinking agents, acting as guests of portions of the chains of certain amphiphilic polymers, for example A-B-A type triblock polymers where A is poly(ethylene oxide) and B is poly(3-hydroxybutyrate), as described in patent WO2004009664 of Xu and Xiping.

To fix the cyclodextrins, as chemically bonded components, to the hydrogels monomer derivatives (vinyl, acrylic, methacrylic derivatives) of cyclodextrin can be used during the synthesis step (Lee et al., J. Appl. Polym. Sci. 80: 438-446, 2001). The materials obtained by these processes have numerous applications in the food, cosmetic and pharmaceutical industry (Friedman, Biotechnol. Food Ingredients, 327-347, 1991). The capacity of cyclodextrin units to form inclusion complexes is not affected by their fixing in hydrogels, provided that their cavities are not blocked. Since the cyclodextrins are immobilized inside the polymer matrix, the delivery rate of the guest drug depends on its affinity for the cavity and also on the diffusion coefficient of the free drug through the matrix (Liu et al, Macromol. Biosci. 4: 729-736, 2004).

Cyclodextrins can also be covalently bonded to a previously formed polymer structure or give rise themselves to crosslinked systems. Processes for polymerizing or crosslinking cyclodextrins in the presence or in the absence of different polymers using epichlorohydrin (Fundueanu et al., J. Chromatogr. B 791: 407-419, 2003) or derivatives of isocyanates (Hishiya et al., Macromolecules 32: 2265-2269, 1999) as crosslinking agents have been described.

The polymer matrices incorporating cyclodextrins, covalently bonded together or to the chains of the polymer components, are of great potential interest as a basis for the creation of polymer materials with specific molecule recognition capacity by applying molecular imprinting techniques (Asanuma et al., Anal. Chim. Acta 435: 25-33, 2001; Alvarez-Lorenzo and Concheiro, J. Chromatogr. B 804: 231-245, 2004). The resulting materials, known as materials with molecular imprinting or imprinted materials, have a suitable spatial distribution so that each cyclodextrin unit can specifically interact with a part of the drug molecule, making the formation of receptors that can recognize it with a high degree of selectivity possible. Polymer lattices have been able to be synthesized by using this process, which lattices have an affinity for peptide and antibiotic drugs with a far more complex structure than that of conventional gels (Asanuma et al., Anal. Chim. Acta 435: 25-33, 2001).

Cellulose ethers are a group of polymers that are soluble in water and/or in organic solvents with very different applications in the pharmaceutical industry (Doelker, Water swollen cellulose derivatives in pharmacy. In Hydrogels in Medicine and Pharmacy, Vol. 2 (Peppas, ed.), CRC Boca Raton, Florida, 1987, pp. 115-160; Doelker, Adv. Polym. Sci. 107: 200-265, 1993). Structurally, they are alkyl modifications of cellulose, resulting from substituting part of the hydrogen atoms of the hydroxyl groups of the anhydrous glucose units with alkyl groups. Each derivative is characterized by its degree of substitution (DS) and its molar substitution (MS). DS indicates the mean number of substituted hydroxyl groups in the anhydrous glucose unit. If the substituents are hydroxyalkyl groups which in turn undergo substitution reactions, side chains can be formed. The MS value reports on the mean number of alkylating reactive molecules that have reacted with each anhydroglucopyronose unit. The MS/DS ratio provides a measurement of the length of the side chains. The nature, the number and the distribution of the substituents conditions, to a large extent, the solubility and the viscosifying capacity of cellulose ethers. The introduction of a polar substituent group (carboxyl or hydroxyl) into glucopyranose increases its solubility in water, which decreases, in contrast, when hydrophobic ether groups predominate.

A number of varieties of cellulose ethers are commercially available. These varieties have different nominal viscosities, DS and molecular weights and different physical properties. Certain cellulose ethers, such as methylcellulose, hydroxypropylcellulose, hydroxypropylmethylcellulose or carboxymethylcellulose, are marketed with a suitable purity for pharmaceutical uses and are "generally recognized as safe" (GRAS) products by the Food and Drug Administration (FDA) (Savage, Ethers. In Cellulose and Cellulose Derivatives (Bikales and Segal, Eds.) Wiley-Interscience, London, 1971, pp. 785-809). The United States Pharmacopoeia 27/National Formulary 22 (2004) and the Real Farmacopea Española *(*Royal Spanish Pharmacopoeia) 2nd edition (2002) contain monographs dedicated to these products. These cellulose ethers are used to prepare a large variety of dosage forms, such as suspensions, emulsions, gels, pellets or tablets (Alderman, Int. J. Pharm. Tech. & Prod. Mfr. 5:1-9, 1984; Vázquez et al., Drug Dev. Ind. Pharm. 18: 1355-1375, 1992).

Guar gums are extracted from the endosperm of seeds of certain plants of the family of legumes, such as *Cyamopsis tetragonolobus.* They are galactomannans resulting from the linear chain formation of β-D-mannose units bonded at (1-4), with branches formed by a single α-D galactose bonded at α (1-6). Guar gums are readily dispersed in hot or cold water. Along with the different guar gum varieties, derivatives such as hydroxypropyl guar gum or carboxymethyl hydroxypropyl guar gum, which are obtained by chemical semi-synthesis processes or by depolymerization (Freeland et al., Cosmet. Toilet. 99: 83-87, 1984), are available on the market.

The chemical crosslinking of cellulose ethers or of guar gums allows obtaining hydrogels having very different applications. The degree of swelling of some of these hydrogels can experience very abrupt changes according to the composition or the temperature of the medium, which makes them quite interesting for developing stimuli-sensitive systems or systems that can release active substances in specific places (Anbergen and Oppermann, Polymer 31: 1854-1858, 1990; Rodríguez et al., J. Controlled Rel. 86: 253-265, 2003).

Different crosslinking agents have been proposed to prepare hydrogels from polysaccharides: trisodium trimetaphosphate has been used to prepare starch or guar gum hydrogels (Gliko-Kabir et al, J. Controlled Rel. 63: 129-134, 2000); borax allows obtaining, in a basic medium, guar gum or hydroxypropylcellulose hydrogels (Shao et al., Macromolecules 33: 19-25, 2000); and divinylsulfone have been used to crosslink cationic cellulose ethers (Sjöstrom and Piculell, Langmuir 14: 3836-3843, 2001). The "gelling time" parameter is used to characterize the crosslinking process, which parameter is defined as the time necessary for the value of the storage modulus to exceed the value of the loss modulus. The time necessary to complete the crosslinking process is estimated as the time from which the values of the storage and loss moduli are held constant. A crosslinking process for crosslinking cationic cellulose ethers and cationically modified guar gums using ethylene glycol diglycidyl ether has recently been perfected by rheometric techniques. The hydrogels prepared by this latter process have proven to be very useful for incorporating and delivering in a controlled manner non-steroidal antiinflammatory drugs (Rodríguez et al., *op. cited* 2003).

With regard to other crosslinking agents such as borax or divinylsulfone, glycidyl ethers have the advantage of having a very low toxicity. Their wide safety margins, along with the absence of effects at the reproductive and endocrine level, and of carcinogenic effects, make them suitable as components for containers that are kept in prolonged contact with foods (Poole et al., Food Additives & Contaminants 21: 905-919, 2004). Crosslinking agents with glycidyl ether groups (also known as epoxides, oxirane or alkene oxides; Allinger et al., Química Orgánica, 2nd Ed. Reverte S.A., Barcelona, 1988, p. 639) allow obtaining cellulose ether or guar gum hydrogels without needing to previously form derivatives of these polymers having polymerizable groups or to previously modify the structure of the cellulose ether or of the guar gum (Rodriguez et al., *op. cited* 2003). Several examples of substances with two or more glycidyl ether groups in their structure are shown below.

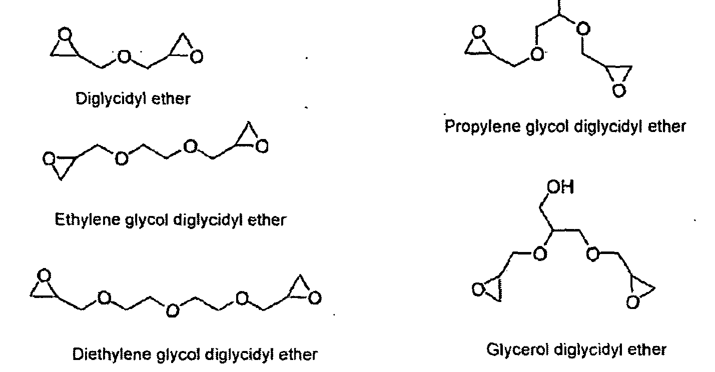

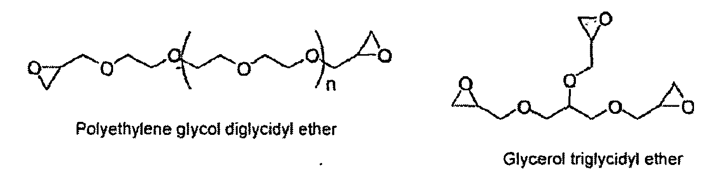

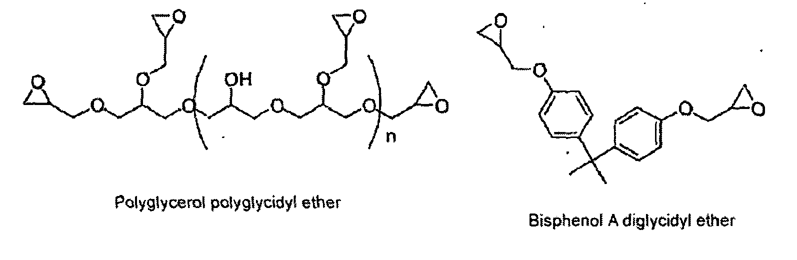

Processes using epoxides to obtain polymeric cyclodextrin derivatives have also been described. US patent 3,420,788 describes a process for obtaining insoluble inclusion resins based on cyclodextrins and polymers insoluble in water, including cellulose derivatives insoluble in water, to be used in powder form in chromatographic separations. Patent JP63012490B describes a process for preparing cyclodextrin particles for chromatographic filler from previously polymerized cyclodextrins using different crosslinking agents, including 1,4-butanediol diglycidyl ether, which are dispersed in an organic solvent for the purpose of obtaining the particles after a second crosslinking process. US patent 4,357,468 describes a process for binding individual units of cyclodextrins to cellulose chains, using, among others, diepoxide agents, to obtain non-crosslinked absorbent materials, useful as cigarette filters, in water purification or for molecular separation. US patent 4,535,152 describes a process in which epoxides are used with ionizable groups in their structure for preparing non-crosslinked chains soluble in water, formed by 2-10 cyclodextrin units, having ionic groups, for the purpose of their use in colloid stabilization or in ion exchange chromatography. British patent GB 2,224,507 describes a process for the preparation of gelatin and cyclodextrin copolymers, using epichlorohydrin or butylene diglycidyl ether as a polymerization agent. US patent 6,048,736 describes a process for covalently binding drugs to individual cyclodextrin units or incorporated into polymer structures. The drug-cyclodextrin bonds and the cyclodextrin bonds to one another are formed by using crosslinking agents which give rise to biodegradable bonds. The delivery of the drug occurs specifically in the places in which these bonds are cleaved. Patent document US2001/0021703 describes a process for crosslinking micelles of amphiphilic cyclodextrins, formed from dimers, trimers and polymers of cyclodextrins using diglycidyl and triglycidyl ethers. The incorporation of the drug is carried out before forming the micelles and their delivery takes place only once the bonds between the cyclodextrin units degrade, the crosslinking agent acting as the agent in charge of controlling the delivery. Patent EP 0,387,681 describes a process for preparing ammonium salts of polycyclodextrins which are soluble in water, to be used as hypocholesteremic agents. The process includes the crosslinking of cyclodextrins using different crosslinking agents and the subsequent esterification of the crosslinked cyclodextrins to obtain the ammonium salt.

### Description of the Invention

The process for obtaining hydrogels from cyclodextrins, from cyclodextrins and cellulose ethers, or from cyclodextrins and guar gums, crosslinked with molecules containing two or more glycidyl ether groups, does not require previously obtaining a cyclodextrin derivative or monomer having polymerizable groups nor does it require the prior modification of the structure of the cellulose ether or of the guar gum either.

To carry out the process cyclodextrins, cyclodextrins and cellulose ethers, or cyclodextrins and guar gums, are used. Cyclodextrin, cellulose ether or guar gum derivatives can also be used. Molecules having two or more glycidyl ether groups in their structure, for example, diglycidyl ether, ethylene glycol diglycidyl ether, diethylene glycol diglycidyl ether, polyethylene glycol diglycidyl ether, polyglycerol polyglycidyl ether, propylene glycol diglycidyl ether, glycerol diglycidyl ether, glycerol triglycidyl ether, or bisphenol A diglycidyl ether, are used as crosslinking agents which are able to react simultaneously with the hydroxyl, amino or carboxyl groups of two or more cyclodextrin molecules, or of a cyclodextrin and a cellulose ether, or of a cyclodextrin and a guar gum. The resulting materials have a hydrogel-type three-dimensional structure and can incorporate, without being dissolved, high proportions of water giving rise to viscoelastic systems.

Any cyclodextrin or any of its derivatives can be used for the process. Examples of cyclodextrins are natural cyclodextrins, α-, β- and γ- cyclodextrin, and other cyclodextrins formed by more than eight units of α-1,4-glucopyranose, known as large cyclodextrins, as well as their derivatives, some of which are included in Table 1.

**Table 1.- Examples of α-, β- and γ- cyclodextrin derivatives.**

| Type of derivative | α-Cyclodextrin | β-Cyclodextrin | γ-Cyclodextrin |
|---|---|---|---|
| Alkylated | Methyl- Butyl- | Methyl- Ethyl- Butyl- | Methyl- Butyl- Pentyl- |
| Hydroxyalkylated | 2-hydroxypropyl- | Hydroxyethyl- 2-hydroxypropyl- 2-hydroxybutyl- | Hydroxyethyl- 2-hydroxypropyl- |
| Esterified | Acetyl- Succinyl | Acetyl- Propionyl- Butyryl- Succinyl- Benzoyl- Palmityl- Toluene sulfonyl | Acetyl- Succinyl- |
| Esterified and alkylated | | Acetyl methyl- Acetyl butyl- | |
| Branched | Glucosyl- Maltosyl- | Glucosyl- Maltosyl- | Glucosyl- Maltosyl- |
| Ionic | Carboxymethyl ether- Phosphate ester- | Carboxymethyl ether- Carboxymethyl ethyl- Phosphate ether- 3-trimethylammonium- 2-hydroxypropyl ether- Sulfobutyl ether- | Carboxymethyl ether- Phosphate ester- |
| Polymerized | Simple carboxymethyl- polymers | Simple carboxymethyl- polymers | Simple carboxymethyl- polymers |

Cellulose ether is understood as any ionic or non-ionic cellulose ether. Examples of cellulose ethers are: methylcellulose (MC), hydroxyethylmethylcellulose (HEMC), hydroxypropylcellulose (HPC), hydroxypropylmethylcellulose (HPMC), hydroxyethylcellulose (HEC), ethylhydroxyethylcellulose (EHEC), sodium carboxymethylcellulose (CMCNa), quaternary ammonium salts of hydroxyethylcellulose with a trimethylammonium substituent (Polyquaternium 10), hydroxyethylcellulose and dimethyldiallylammonium chloride copolymers (Polyquaternium A). Any variety of guar gum, any modified guar gum or any of its derivatives, is suitable for the process. Examples of guar gum derivatives are its hydroxypropylated or carboxyhydroxypropylated derivatives, its cationic derivatives (Ecopol) and the products resulting from the depolymerization of guar gums.

To carry out the process, the cyclodextrin solution in water or in hydroalcoholic medium is first prepared and the necessary volume or a solution of HCl or another acidifying agent, or of NaOH or of another alkalizing agent is added to adjust the pH to a suitable value for the crosslinking process to take place (either acid, neutral or alkaline). The cyclodextrin can also be directly dissolved in a medium with the suitable pH. To prepare cyclodextrin and cellulose ether, or cyclodextrin and guar gum, hydrogels the incorporation of the cellulose ether or of the guar gum to water or to the hydroalcoholic medium can be carried out before or after dissolving the cyclodextrin. In any of the two cases, the resulting solution is homogenized using a magnetic or mechanical stirrer and if necessary, applying ultrasound. The suitable amount of crosslinking agent, in solid or liquid state, or in solution, is then incorporated with stirring.

To obtain cyclodextrin hydrogels or their derivatives, the proportion of cyclodextrin or of the cyclodextrin derivative can be comprised between 1 and 95% of the total of the hydrogel components excluding water, typical values being those comprised between 4% (w/w) and 70% (w/w), and the proportion of the crosslinking agent is comprised between 99% and 5% of the total of the hydrogel components excluding water, typical values being those comprised between 96% (w/w) and 30% (w/w).

To obtain cyclodextrin hydrogels or their derivatives and cellulose ethers or their derivatives, the proportion of cyclodextrin or of the cyclodextrin derivative is comprised between 1% and 95% of the total of the hydrogel components excluding water, typical values being those comprised between 4% (w/w) and 70% (w/w); the proportion of cellulose ether or of the cellulose ether derivative is comprised between 0.05% and the 95% of the total of the hydrogel components excluding water, typical values being those comprised between 0.1 % (w/w) and 20 % (w/w); and the proportion of the crosslinking agent is comprised between 98.95% and 4% of the total of the hydrogel components excluding water, typical values being those comprised between 96 % (w/w) and 30 % (w/w).

To obtain cyclodextrin hydrogels or their derivatives and guar gums or their derivatives, the proportion of cyclodextrin or of the cyclodextrin derivative is comprised between 1% and the 95% of the total of the hydrogel components excluding water, typical values being those comprised between 4 % (w/w) and 70 % (w/w); the proportion of guar gum or of the guar gum derivative can be comprised between 0.05% and 95% of the total of the hydrogel components excluding water, typical values being those comprised between 0.1 % (w/w) and 20 % (w/w); and the proportion of the crosslinking agent is comprised between 98.95% and 4% of the total of the hydrogel components, excluding water, typical values being those comprised between 96 % (w/w) and 30 % (w/w).

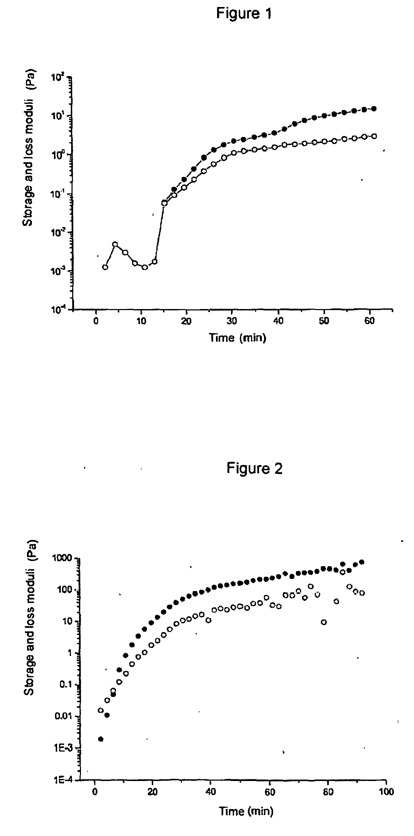

The obtained solution is homogenized, transferred to a suitable mold and allowed to rest at a controlled temperature between 0 and 100°C, for the necessary time for the crosslinking to be completed. To establish the necessary time for achieving hydrogel formation, oscillating shear rheometry can be applied using samples of the solutions of cyclodextrins and crosslinking agent, of cyclodextrins, cellulose ethers and crosslinking agent, or of cyclodextrins, guar gums and crosslinking agent. This technique allows estimating the gelling time and the necessary time for completing the crosslinking process (Figure 1). The gelling time parameter value can be comprised between 1 second and 12 hours, typical values being between 10 minutes and 2 hours. The necessary time for completing crosslinking can be comprised between 3 seconds and 24 hours, typical values being between 10 minutes and 6 hours.

The hydrogel, once formed, is removed from the mold and immersed, in order to wash it, in a container with aqueous or hydroalcoholic medium, until eliminating the unreacted substances. The washing process is considered to have ended when the absorbance of the washing medium is less than 0.001 in the entire wavelength interval comprised between 190 and 800 nm. The washing times are usually comprised between 1 hour and 3 days.

Once washed, the hydrogels are divided into portions with suitable size and shape and are used as they are upon removing them from the washing medium or after subjecting them to drying. A vacuum oven or an airstream, at a temperature comprised between 30 and 80°C. can be used to dry them. The hydrogels can also be dried by lyophilization.

The drug or the active substance can then be incorporated to the hydrogel by direct immersion in a solution or a suspension of the drug or of the active substance, at a temperature comprised between 0 and 100°C and at atmospheric pressure, with or without the aid of ultrasound. The incorporation can also be carried out in an autoclave at a temperature comprised between 100 and 130°C.

The obtained compositions, with or without incorporated drugs or active substances, can be used as such or as base components of pharmaceutical forms, medicinal products and plant protection products for the treatment of pathological or physiological conditions in humans, animals and plants, such as transdermal forms, transmucosal forms, such as for example, buccal, oral, rectal, ocular, nasal, otic or vaginal dosage forms, and parenteral implants. They can also be used as sequestering agents of biological or toxic substances in live organisms, for example, cholesterol, glucose or biliary acids, or in the environment. The invention also covers their use in cosmetics.

### Advantages and improvements with respect to already existing processes and materials

The process object of the invention leads to obtaining compositions with a hydrogel-type three-dimensional structure with a high affinity for water, but which do not dissolve, and which are provided with a high capacity for incorporating drugs, active substances, biological or toxic molecules with very different structures and physical and chemical properties, forming inclusion complexes with the cyclodextrins forming part of their structure. The capacity for incorporating a drug or an active substance of the compositions containing cyclodextrins and cellulose ethers or their derivatives, or cyclodextrins and guar gums or their derivatives, is comprised between 50 and 5000% (w/w) of that of obtained hydrogels, in the same conditions, with cellulose ethers or their derivatives, or guar gums or their derivatives, without cyclodextrins. Therefore, in these new compositions, the capacity for incorporating substances is intensely enhanced with respect to the hydrogels that are exclusively prepared with cellulose ethers or their derivatives, or guar gums or their derivatives. This involves a very important improvement for the use of the compositions as drug or active substance carriers in humans, animals or plants, or as "trap" systems of biological or toxic substances in live organisms or of water pollutants.

The compositions have, in the hydrated state, viscoelastic properties. The compositions are also very suitable for controlling the delivery of drugs or active substances, which are incorporated by immersing, in their solutions or in their suspensions, of the hydrogel once it has been synthesized, by diffusion of formation of inclusion complexes and/or non-covalent bonds. The compositions provide different delivery rates depending on their qualitative and quantitative compositions and on the physical and chemical properties of the drug, especially of its water-solubility and its affinity for the cyclodextrin cavity. For a water-soluble drug or active substance with a constant of affinity for hydroxypropyl-beta-cyclodextrin equal to 115 M⁻¹, typical delivery percentage values are 50% after 2 hours, 80% after 4 hours and 100% after 8 hours. For a lipophilic drug or active substance with a constant of affinity for hydroxypropyl-beta-cyclodextrin equal to 17000 M⁻¹, typical delivery percentage values are 20% after 2 hours, 50% after 8 hours and 70% after 48 hours.

They may also be useful for directing drugs to specific areas in live organisms by means of changes associated to the environmental conditions, in the swelling degree of the hydrogel or in the affinity of the drug for the hydrogel components. All these characteristics can be modulated through a suitable selection of the variety and/or of the proportion of cyclodextrin or cyclodextrins and of the cellulose ethers or their derivatives, or guar gums or their derivatives accompanying it or them. The low or nil toxicity of the cyclodextrins, the cellulose ethers; the guar gums and their derivatives, and the glycidyl ether crosslinking agents make it possible to use the resulting compositions as components of pharmaceutical forms, of cosmetic preparations or "trap" systems for capturing molecules from live organism or from the environment, without posing biocompatibility or environmental impact problems. Furthermore, the process takes place in conditions that generally do not compromise the stability of the drugs or the active substances, and does not generate waste involving environmental pollution risks.

### Commercial applications

The compositions can be used in very different fields, such as the development of pharmaceutical forms and drug and active substance releasing systems for immediate delivery or which can control the delivery or direct the drug to specific areas, cosmetic preparations or plant protection product or active substance releasing systems, to be used in humans, animals and plants. They can also be used to develop systems which can sequester toxic substances or molecules produced by live organisms in the biological environment. They can also be applied in the removal of pollutants in water or other liquid media.

### Examples of the Invention

Several examples showing how to obtain hydrogels using cyclodextrins or their derivatives, cyclodextrins or their derivatives and cellulose ethers or their derivatives, cyclodextrins or their derivatives and guar gums and their derivatives are included below. Examples of the monitoring of the formation process of several hydrogels by means of oscillating rheometry techniques are also included. Examples of the preparation of compositions including drugs and controlling their delivery are also included. An example testing the formation of inclusion complexes of a drug with the cyclodextrin units incorporated in the hydrogel is also included.

### Example 1. Process for obtaining a hydrogel based on γ-cyclodextrin

A 20% (w/w) γ-cyclodextrin solution in 0.2M NaOH was prepared. 2 mL of a 50% (w/w) ethylene glycol diglycidyl ether in water was added to 5 mL of this solution, such that the final concentration of the crosslinking agent was 14.28%. The mixture was subjected to stirring for 1 minute to achieve its complete homogenization. It was then transferred to a test-tube with an inner diameter of 0.8 cm, and was allowed to rest at 50°C for 12 hours, to complete the formation of the hydrogel. After this time, the hydrogel was removed from the mold and washed by immersion in 0.01 M HCl and distilled water, remaining 12 hours in each medium. The proportion of γ-cyclodextrin in the hydrogel is 58.34 % (w/w) and the proportion of the crosslinking agent is 41.66 % (w/w) of the total of the hydrogel components excluding water.

### Example 2. Process for obtaining a hydrogel based on γ-cyclodextrin and hydroxypropylmethylcellulose (HPMC) and process monitoring.

A 20% (w/w) γ-cyclodextrin solution in 0.2M NaOH was prepared and hydroxypropylmethylcellulose (HPMC) with a nominal viscosity of 4000 cPs was incorporated to it in the necessary amount to reach a final concentration of cellulose ether of 0.4% (w/w). 2 mL of a 50% (w/w) ethylene glycol diglycidyl ether in water was added to 5 mL of this solution, such that the final concentration of the crosslinking agent was 14.28%. The mixture was subjected to stirring for 1 minute to achieve its complete homogenization. A sample of the mixture was immediately transferred to the Peltier plate of a controlled-stress torsional rheometer and was tested at 50°C in oscillating mode, applying a shear force of 0.1 Pa at a frequency of 0.1 rad/s, to record the evolution over time of the storage and loss moduli (Figure 2). The gelling time was approximately 15 minutes and the necessary time for completing the crosslinking process was approximately 45 minutes. The remaining mixture was transferred to a test-tube with an inner diameter of 0.8 cm, and was allowed to rest at 50°C for 12 hours. After this time, the hydrogel was removed from the mold and washed by immersion in 0.01 M HCl and distilled water, remaining 12 hours in each medium. The proportion of γ-cyclodextrin in the hydrogel is 57.67% (w/w), the proportion of HPMC is 1.15 % (w/w) and the proportion of the crosslinking agent is 41.66 % (w/w) of the total of the hydrogel components excluding water.

### Example 3. Process for obtaining a hydrogel based on hydroxypropyl-β-cyclodextrin and sodium carboxymethylcellulose (CMCNa), and process monitoring.

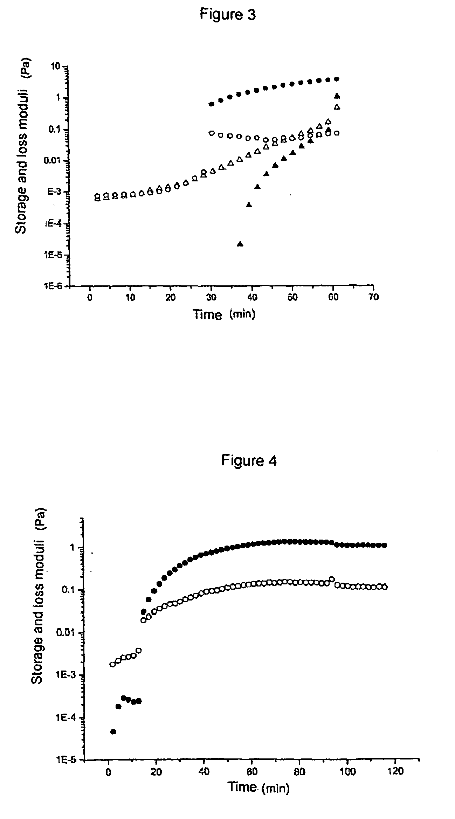

20% (w/w) hydroxypropyl-β-cyclodextrin solutions in 0.2M NaOH or in 0.2M KOH were prepared. Sodium carboxymethylcellulose with a nominal viscosity of 400-800 cps was incorporated to 5 mL aliquots of these solutions in the necessary amounts to reach a final concentration of the cellulose ether of 0.4% (w/w) or of 0.8% (w/w). 2 mL of a 50% (w/w) ethylene glycol diglycidyl ether solution in water (final concentration of 14.28%) were then added to each solution. The mixtures were subjected to stirring for 1 minute to achieve their complete homogenization. Samples were immediately taken from each mixture, transferred to the Peltier plate of a controlled-stress torsional rheometer and tested at 50°C in oscillating mode, applying shear force of 0.1 Pa at a frequency of 1 rad/s, to register the evolution over time of the storage and loss moduli (Figure 3). The gelling time was, in all cases, less than 1 hour and the necessary time to complete the crosslinking process was 6 hours when NaOH was used and 12 hours when KOH was used as an alkalizing agent.

The remaining portions of the mixtures were transferred to test-tubes with an inner diameter of 0.8 cm and were allowed to rest at 50°C for 12 hours. After this time, the hydrogels were removed from the molds and washed by immersion in 0.01 M HCI and distilled water, remaining 12 hours in each medium. The proportion of hydroxypropyl-β-cyclodextrin in these hydrogels is comprised between 57.0% and 57.7% (w/w), the proportion of sodium carboxymethylcellulose is comprised between 1.15 % and 2.29 % (w/w) and the proportion of the crosslinking agent is comprised between 41.18 % and 40.71% (w/w) of the total of the hydrogel components excluding water.

An increase in the concentration of sodium carboxymethylcellulose or the use of NaOH instead of KOH as an alkalizing agent, allowed increasing the values of both moduli, reducing the gelling time and giving rise to more viscoelastic hydrogels.

### Example 4. Process for obtaining a hydrogel based on crystalline methyl-β-cyclodextrin and hydroxypropylmethylcellulose (HPMC), and process monitoring.

A 15% (w/w) methyl-β-cyclodextrin (CRISMEB) solution in 0.1M HCI was prepared and hydroxypropylmethylcellulose (HPMC) with a nominal viscosity of 4000 cPs was added to it in the necessary amount to reach a final concentration of cellulose ether of 0.4% (w/w). 2 mL of a 50% (w/w) ethylene glycol diglycidyl ether in water was then added to 10 mL of this solution, such that the final concentration of the crosslinking agent was 14.28%. The mixture was subjected to stirring for 1 minute to achieve its complete homogenization. A sample of the mixture was immediately transferred to the Peltier plate of a controlled-stress torsional rheometer and was tested at 50°C in oscillating mode, applying a shear force of 0.1 Pa at a frequency of 1 rad/s, to record the evolution over time of the storage and loss moduli (Figure 4). The gelling time was approximately 20 minutes and the necessary time to complete the crosslinking process was approximately 45 minutes. The remaining mixture was transferred to a test-tube with an inner diameter of 0.8 cm and was allowed to rest at 50°C for 12 hours. After this time, the hydrogel was removed from the mold and washed by immersion in 0.01 M NaOH and distilled water, remaining 12 hours in each medium. The proportion of methyl-β-cyclodextrin in the hydrogel is 50.54% (w/w), the proportion of HPMC is 1.35 % (w/w) and the proportion of the crosslinking agent is 48.11% (w/w) of the total of the hydrogel components excluding water.

### Example 5. Process for obtaining a hydrogel based on hydroxypropyl-β-cyclodextrin and cationically modified guar gum, and process monitoring.

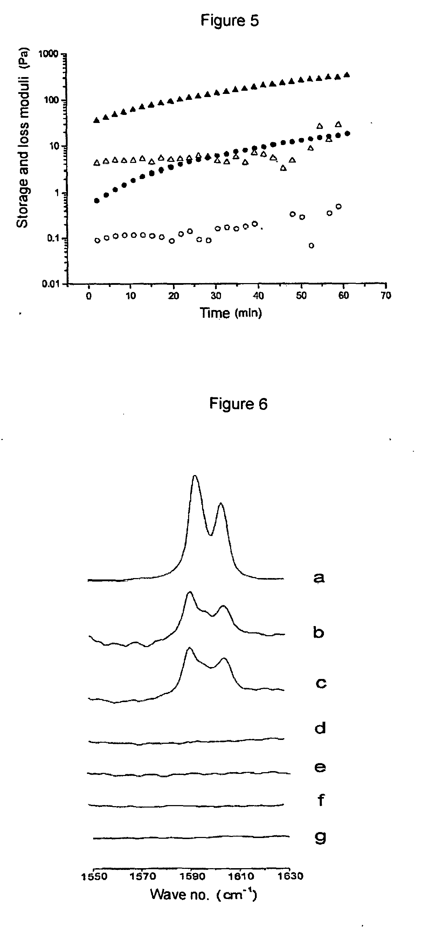

20% (w/w) hydroxypropyl-β-cyclodextrin solutions in 0.2M NaOH or in 0.2M KOH were prepared. Cationically modified guar gum (Ecopol E-261-S) with a molecular weight of 200000 Da was added to 5 mL aliquots of these solutions, in the necessary amounts to reach a final concentration of guar gum of 0.4% (w/w) or of 0.8% (w/w). 2 mL of 50% (w/w) ethylene glycol diglycidyl ether solution in water were then added to each solution, such that the final concentration of crosslinking agent was 14.28% in all cases. The mixtures were subjected to magnetic stirring for 1 minute to achieve their complete homogenization. Samples were immediately taken from each mixture, transferred to the Peltier plate of a controlled-stress torsional rheometer and tested at 50°C in oscillating mode, applying a shear force of 0.1 Pa at a frequency of 1 rad/s, to register the evolution over time of the storage and loss moduli (Figure 5). The gelling time was, in all the cases, less than 1 hour and the necessary time to complete the crosslinking process was 6 hours.

The remaining portions of the mixtures were transferred to test-tubes with an inner diameter of 0.8 cm and were allowed to rest at 50°C for 12 hours. After this time, the hydrogels were removed from the molds and washed by immersion in 0.01 M HCI and distilled water, remaining 12 hours in each medium. The proportion of hydroxypropyl-β-cyclodextrin in these hydrogels is comprised between 57.0% and 57.7% (w/w), the proportion of cationically modified guar gum is comprised between 1.15 % and 2.29 % (w/w) and the proportion of the crosslinking agent is comprised between 41.18 % and 40.71% (w/w) of the total of the hydrogel components excluding water. An increase in the concentration of cationically modified guar gum allowed increasing the values of both moduli, reducing the gelling time and giving rise to more viscoelastic hydrogels.

### Example 6. Process for obtaining a composition based on hydroxypropyl-β-cyclodextrin and a composition based on hydroxypropyl-β-cyclodextrin and hydroxypropylmethylcellulose (HPMC), incorporating diclofenac sodium and delivering it in a controlled manner

A 20% (w/w) hydroxypropyl-β-cyclodextrin solution in 0.2M NaOH was prepared. The necessary amounts of hydroxypropylmethylcellulose (HPMC) with a nominal viscosity of 4000 cPs were added to 5 mL aliquots of this solution, so as to reach concentrations of cellulose ether comprised between 0.2% (w/w) and 1.0% (w/w). 2 mL of a 50% (w/w) ethylene glycol diglycidyl ether solution in water were added to each of the resulting solutions, such that the final concentration of crosslinking agent was 14.28% in all cases. The mixtures were homogenized using a magnetic stirrer, transferred to test-tubes with an inner diameter of 0.8 cm and allowed to rest at 50°C for 12 hours, to complete hydrogel formation. After this time, the hydrogels were removed from the molds and immersed in distilled water. After 12 hours, they were transferred to containers with 0.01 M HCl, where they were kept for another 12 hours. They were finally immersed for another 12 hours in distilled water. Each hydrogel was divided into disc-shaped portions, with a diameter of 8 mm and a thickness of 5 mm. Three hydrogel discs were directly placed in 10 mL vials of 0.1% (w/w) or 0.5% (w/w) diclofenac solution for two days. Another three hydrogel discs were subjected to drying in an oven at 40°C before they were introduced in the vials with the diclofenac solution.

To determine the amount of diclofenac incorporated to each hydrogel disc, the absorbance of the medium at 276 nm was measured before and once the incorporation process was completed. Table 2 shows by way of example the diclofenac contents in hydrogel discs with a different composition, to which the drug was incorporated after subjecting them to drying in an oven.

**Table 2. Amount of diclofenac incorporated by hydroxypropyl-β-cyclodextrin (HPβCD) hydrogels or hydroxypropyl-β-cyclodextrin and hydroxypropylmethylcellulose (HPMC) hydrogels, crosslinked with ethylene glycol diglycidyl ether (EGDE).**

| Starting solutions | | Hydrogel composition | | | Amount of diclofenac incorporated (mg/g of dry hydrogel) | |
|---|---|---|---|---|---|---|
| HPβCD (%, w/w) | HPMC (%, w/w) | HPβCD (%, w/w) | HPMC (%, w/w) | EGDE (%, w/w) | Hydrogels immersed in 0.1% drug solution | Hydrogels immersed in 0.5% drug solution |
| 20 | 0 | 58.34 | 0 | 41.66 | 11.08 | 424.13 |
| 20 | 0.2 | 58.00 | 0.58 | 41.42 | 10.31 | 285.54 |
| 20 | 0.4 | 57.67 | 1.15 | 41.18 | 8.95 | 120.96 |
| 20 | 0.6 | 57.34 | 1.72 | 40.94 | 14.23 | 132.72 |
| 20 | 0.8 | 57.01 | 2.28 | 40.71 | 16.97 | 120.85 |
| 20 | 1.0 | 56.69 | 2.83 | 40.48 | 14.94 | 169.51 |

After incorporating diclofenac, the hydrogels were dried in an air oven at 40°C. Their Raman spectra were registered in a Fourier transform IR spectrophotometer. Figure 6 shows the IR spectra, in which shifts of approximately 2 cm⁻¹ to lower wavelength values and changes in the intensity of the bands were located between 1500 and 1650 cm⁻¹, which are characteristic of the formation of a complex between the drug and the cyclodextrin (Iliescu et al., Eur. J. Pharm. Sci. 22: 487-495, 2004), can be observed. These facts confirm that the drug is incorporated to the hydrogel, forming inclusion complexes with its cyclodextrin units.

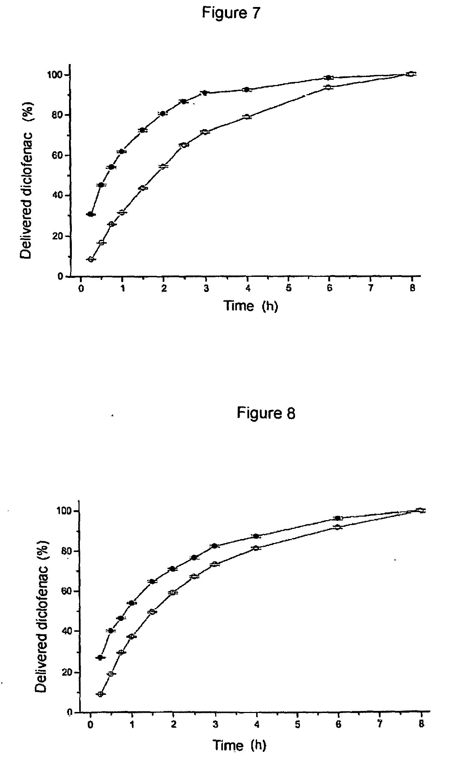

Figures 7 and 8 show the delivery profiles of diclofenac from hydrogels prepared with hydroxypropyl-β-cyclodextrin, without hydroxypropylmethylcellulose or with 0.4% (w/w) of hydroxypropylmethylcellulose, respectively. The test was carried out using 25 mL of water without stirring. All the compositions controlled the release process up to a maximum of 8 hours.

### Example 7. Process for obtaining compositions based on hydroxypropyl-β-cyclodextrin and hydroxypropylmethylcellulose (HPMC), incorporating estradiol and delivering it a controlled manner.

15%, 20% and 25% (w/w) hydroxypropyl-β-cyclodextrin solutions in 0.2M NaOH were prepared. The necessary amount of hydroxypropylmethylcellulose (HPMC) with a nominal viscosity of 4000 cPs to reach a final concentration of cellulose ether of 0.4% (w/w), and the necessary volume of 50% (w/w) ethylene glycol diglycidyl ether solution in water to reach a final concentration of the crosslinking agent of 14.28% (w/w) was added to each of them. The mixtures were homogenized using a magnetic stirrer, transferred to test-tubes with an inner diameter of 0.8 cm, and allowed to rest at 50°C for 12 hours, to complete hydrogel formation. After this time, the hydrogels were removed from the molds and immersed in distilled water. After 12 hours, they were transferred to containers with 0.01 M HCI, where they were kept for another 12 hours. They were finally immersed for another 12 hours in distilled water and kept in this medium until the time of incorporating estradiol. Each hydrogel was divided into disc-shaped portions, with a diameter of 8 mm and a thickness of 5 mm. The proportion of hydroxypropyl-β-cyclodextrin in these hydrogels is comprised between 50.54% and 63.00% (w/w), the proportion of HPMC is comprised between 1.35% and 1.00% (w/w) and the proportion of the crosslinking agent is comprised between 48.11% and 35.99% (w/w) of the total of the hydrogel components excluding water.

Estradiol was incorporated to each of these portions by immersing them, in suitable containers, in 10 mL of 0.2% (w/w) drug suspension. The containers were closed and taken to an autoclave where they were subjected to a heating cycle (121°C, 16 minutes), then keeping them for seven days in a thermostatted chamber at 25°C. The amount of estradiol incorporated was determined by immersing the hydrogels in 15 mL of an aqueous 0.3% (w/w) sodium dodecyl sulfate solution for 14 days, and assessing by direct spectrophotometry the amount of drug delivered to the medium, which was comprised between 550 and 700 micrograms estradiol per hydrogel disc.

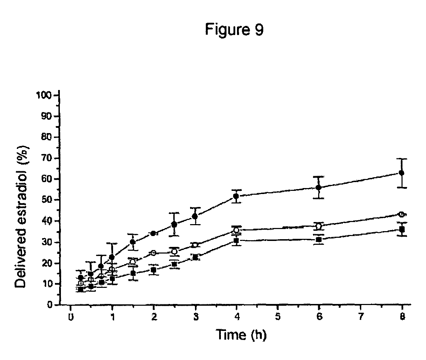

The hydrogels with the incorporated estradiol were immersed in 15 mL of an aqueous 0.3% (w/w) sodium dodecyl sulfate solution and the amount of drug delivered at different times was assessed by ultraviolet spectrophotometry at 280 nm (Figure 9). The delivery profiles obtained show that the compositions control the delivery process for more than one week.

### Description of the Drawings

Figure 1. Evolution over time of the values of the storage (•) and loss (o) modulus during the crosslinking of hydroxypropyl-β-cyclodextrin (20%, w/w) and hydroxypropylmethylcellulose (0.4% w/w) with ethylene glycol diglycidyl ether (14.28%, w/w) at 50°C.
Figure 2. Evolution over time of the values of the storage (•) and loss (o) modulus during the crosslinking of γ-cyclodextrin (20%, w/w) and hydroxypropylmethylcellulose (HPMC) (0.4%, w/w) in 0.2 M NaOH with ethylene glycol diglycidyl ether (14.28%, w/w) at 50°C.
Figure 3. Evolution over time of the values of the storage **(•,▲)** and loss (ο, Δ) modulus during the crosslinking of hydroxypropyl-beta-cyclodextrin (20%, w/w) with carboxymethylcellulose (0.4%, w/w) and ethylene glycol diglycidyl ether (14.28%, w/w) in 0.2M NaOH (•, ο) or in 0.2M KOH (▲, Δ) at 50°C.
Figure 4. Evolution over time of the values of the storage (•) and loss (ο) modulus during the crosslinking of methyl-β-cyclodextrin (20%, w/w) and hydroxypropylmethylcellulose (HPMC) (0.4%, w/w) in 0.1M HCl with ethylene glycol diglycidyl ether (14.28%, w/w) at 50°C.
Figure 5. Evolution over time of the values of the storage (•,▲) and loss (ο, Δ) modulus during the crosslinking of hydroxypropyl-beta-cyclodextrin (20%, w/w) and cationically modified guar gum (0.4%, w/w) in 0.2 M NaOH (•, ο) or 0.8% (w/w) in 0.2M KOH (▲,Δ) with ethylene glycol diglycidyl ether (14.28%, w/w) at 50°C.
Figure 6. FT-Raman spectra in the region 1550-1630 cm⁻¹ of (a) diclofenac, (b) dried hydroxypropylmethylcellulose (HPMC) (0.4%, w/w) and hydroxypropyl beta-cyclodextrin (HPβCD) (20%, w/w) hydrogel with diclofenac incorporated by immersion in the 0.5% (w/w) drug solution, (c) dried HPβCD (20%, w/w) hydrogel with diclofenac incorporated by immersion in the 0.5% (w/w) drug solution, (d) dried HPMC (0.4%, w/w) and HPβCD hydrogel, (e) dried HPβCD (20% (w/w) hydrogel, (f) HPMC, (g) HPβCD.
Figure 7. Delivery profiles of diclofenac from compositions based on hydroxypropyl-beta-cyclodextrin (HPβCD) (20%, w/w) to which the drug has been incorporated by immersion in a 0.1% (w/w) diclofenac sodium solution (Table 2). The compositions were introduced in the delivery medium directly (•) or after drying them in an oven at 40°C (o). The tests were carried out in 25 mL of water, without stirring.
Figure 8. Delivery profiles of diclofenac from compositions based on hydroxypropyl-beta-cyclodextrin (HPβCD) (20%, w/w) and hydroxypropylmethylcellulose (HPMC) (0.4%, w/w), to which the drug was incorporated by immersion in a 0.1% (w/w) diclofenac sodium solution (Table 2). The compositions were introduced in the delivery medium directly (•) or after drying them in an oven at 40°C (ο). The tests were carried out in 25 mL of water, without stirring.
Figure 9. Delivery profiles of estradiol from compositions based on 0.4% (w/w) hydroxypropylmethylcellulose (HPMC) and 15% (w/w) (•), 20% (w/w) (ο) and 25% (w/w) (■) hydroxypropyl-beta-cyclodextrin (HPβCD), with ethylene glycol diglycidyl ether (14.28% w/w), to which the drug was incorporated by immersion in an estradiol solution, autoclaved at 121°C for 16 minutes and storage for seven days in a thermostatted chamber at 25°C. The tests were carried out in 15 mL of a 0.3% (w/w) aqueous sodium dodecyl sulfate solution, without stirring.

## Claims

1. A process for obtaining hydrogels **characterized in that** the hydrogels are formed by:
i. cyclodextrins or their derivatives; or
ii. cyclodextrins or their derivatives, and cellulose ethers or their derivatives; or
iii. cyclodextrins or their derivatives, and guar gums or their derivatives;
iv. and furthermore, using molecules containing two or more glycidyl ether groups in their structure as a crosslinking agent.

2. A process for obtaining hydrogels according to claim 1, **characterized in that** in case i) it comprises the following steps: a) dissolving cyclodextrin or a cyclodextrin derivative in water or a hydroalcoholic medium; b) adjusting the pH of the solution; c) incorporating the crosslinking agent and homogenizing the mixture; d) transferring to a mold; e) maintaining in a thermostatted chamber at a temperature comprised between 0 and 100°C; f) removing from the mold; g) washing the hydrogel; h) dividing into portions with a suitable shape and size.

3. A process for obtaining hydrogels according to claims 1 and 2, **characterized in that** in case ii) it comprises the following steps: a) dissolving cyclodextrin or a cyclodextrin derivative and cellulose ether or a cellulose ether derivative in water or a hydroalcoholic medium; and then steps b) to h) of claim 2

4. A process for obtaining hydrogels according to claims 1 and 2, **characterized in that** in case iii) it comprises the following steps: a) dissolving cyclodextrin or a cyclodextrin derivative and guar gum or a guar gum derivative in water or a hydroalcoholic medium; and then steps b) to h) of claim 2

5. A process for obtaining hydrogels according to claims 1, 2, 3 and 4, **characterized in that** in steps a) and b) the reaction medium is water or a hydroalcoholic medium and has an acid, neutral or alkalin e pH.

6. A process for obtaining hydrogels according to claims 1, 2, 3, 4 and 5, including a step of drying the hydrogels in a vacuum oven or with an airstream, or by lyophilization, between steps g) and h) or after step h).

7. A process for obtaining hydrogels according to the previous claims, **characterized in that** the cyclodextrins or their derivatives are α-, β- and γ-cyclodextrins, cyclodextrins formed by more than eight α-1,4-glucopyranose units, and the methyl-, ethyl-, butyl-, hydroxyethyl-, 2-hydroxypropyl-, 2-hydroxybutyl-, acetyl-, propionyl-, butyryl-, succinyl-, benzoyl-, palmityl-, toluene sulfonyl-, acetyl methyl-, acetyl butyl-, glucosyl-, maltosyl-, carboxymethyl ether-, carboxymethyl ethyl-, phosphate ester-, 3-trimethylammonium-, sulfobutyl ether cyclodextrin derivatives, and cyclodextrin polymers.

8. A process for obtaining hydrogels according to claims 1 and 3, **characterized in that** cellulose ethers or their derivatives are ionic or non-ionic ethers, such as methylcellulose (MC), hydroxyethylmethylcellulose (HEMC), hydroxypropylcellulose (HPC), hydroxypropylmethylcellulose (HPMC), hydroxyethylcellulose (HEC), ethylhydroxyethylcellulose (EHEC), sodium carboxymethylcellulose (CMCNa), quaternary ammonium salts of hydroxyethylcellulose with a trimethylammonium substituent and dimethyl diallylammonium chloride and hydroxyethylcellulose copolymers.

9. A process for obtaining hydrogels according to claims 1 and 4, **characterized in that** guar gums or their derivatives are any guar gum, any modified guar gum or any guar gum derivative, such as any hydroxypropylated or carboxyhydroxypropylated ether, any cationic derivative or any product resulting from its depolymerization.

10. A process for obtaining hydrogels according to claims 1 to 6, **characterized in that** the crosslinking agent is a substance containing two or more glycidyl ether groups in its structure; understanding glycidyl ether as: oxirane, epoxide, or alkene oxide; such as: diglycidyl ether, ethylene glycol diglycidyl ether, diethylene glycol diglycidyl ether, propylene glycol diglycidyl ether, polyethylene glycol polyglycidyl ether, glycerol diglycidyl ether, glycerol triglycidyl ether, polyglycerol polyglycidyl ether and bisphenol A diglycidyl ether.

11. A process for obtaining hydrogels according to claims 1, 2, 7 and 10, **characterized in that** the proportion of cyclodextrin or of the cyclodextrin derivative is comprised between 1% and 95% of the total of the hydrogel components, and the proportion of the crosslinking agent is comprised between 99% and 5%.

12. A process for obtaining hydrogels according to claims 1, 3, 8 and 10, **characterized in that** the proportion of cyclodextrin or of the cyclodextrin derivative is comprised between 1% and 95%; the proportion of cellu lose ether or of the cellulose ether derivative is comprised between 0.05% and 95%; and the proportion of the crosslinking agent is comprised between 98.95% and 4% of the total of the hydrogel components.

13. A process for obtaining hydrogels according to claims 1, 4, 9 and 10, **characterized in that** proportion of cyclodextrin or of the cyclodextrin derivative is comprised between 1% and 95%: the proportion of guar gum or of the guar gum derivative is comprised between 0.05% and 95%; and the proportion of the crosslinking agent is comprised between 98.95% and 4% of the total of the hydrogel components.

14. A process for obtaining hydrogels according to the previous claims, including a step of incorporating the drug or active substance by immersing the wet or previously dried hydrogel in a solution or in a suspension of the drug or of the active substance, at a temperature comprised between 0 and 100°C and at atmospheric pressure, with or without the aid of ultrasound. The incorporation can also be carried out in an autoclave at a temperature comprised between 100 and 130°C.

15. Compositions obtained according to the previous claims.

16. Compositions according to the previous claims, incorporating the hydrogels drugs, active substances or nutrients for their controlled release.

17. Compositions according to the previous clams, **characterized in that** in living organism or in the environment, they capture biological or toxic substances acting as sequestering agents.

18. The use of the compositions according to the previous claims for preparing a medicinal product or plant protection product for the treatment of pathological or physiological conditions in humans, animals and plants.

19. The use of the compositions according to claim 16 for preparing a medicinal product for transdermal, transmucosal, buccal, oral, rectal, ocular, nasal, otic or vaginal administration, or as a parenteral implant.

20. The use of the compositions according to claims 1 to 16 for preparing cosmetics.

21. The use of the compositions according to claim 17 for preparing a sequestering agent of biological or toxic substances to act in living organisms or in the environment.
